# FASCICULE DE BREVET EUROPEEN

(11) **EP 2 413 908 B1**
(45) Date de publication et mention de la délivrance du brevet: **24.08.2016**
(21) Numéro de dépôt: 10711896.0
(22) Date de dépôt: 31.03.2010
(51) Int. Cl.: A61K 9/14, A61K 31/436, A61K 31/445, A61K 9/16, A61K 9/20

(54) **COMPOSITION PHARMACEUTIQUE COMPRENANT UN MACROLIDE IMMUNOSUPPRESSEUR DE LA FAMILLE DES "LIMUS"**
PHARMAZEUTISCHE ZUSAMMENSETZUNG MIT EINEM IMMUNSUPPRESSIVEN MAKROLID DER LIMUS-FAMILIE
PHARMACEUTICAL COMPOSITION CONTAINING A LIMUS FAMILY IMMUNOSUPPRESSIVE MACROLIDE

(30) Priorité: 31.03.2009 FR 0952049
(43) Date de publication de la demande: 08.02.2012
(73) Titulaire: ETHYPHARM, 92210 Saint-Cloud (FR)
(72) Inventeur: DESCHAMPS, Frantz, F-54000 Nancy (FR); HERRY, Catherine, F-27670 Saint-ouen Du Tilleul (FR); JUNG, Jennifer, F-54380 Dieulouard (FR); LEBOEUF, Fabrice, F-54130 Saint Max (FR)
(74) Mandataire: Regimbeau
(86) Numéro de dépôt international: PCT/EP2010/054277
(87) Numéro de publication internationale: WO 2010/112541

(56) Documents cités:
- WO-A-2006/125496
- WO-A-2008/106503
- WO-A-2008/137148
- WO-A1-2008/131131

## Description

La présente invention a pour objet une nouvelle composition pharmaceutique pour administration orale comprenant essentiellement des particules fines cristallines d'une lactone macrocyclique appartenant à la classe thérapeutique des immuno-suppresseurs agissant sur les immunophilines, en particulier un macrolide immunosuppresseur de la famille des « limus » et un ou plusieurs excipients pharmaceutiquement acceptables.

Plus particulièrement, la présente invention concerne des formulations excipients/ macrolide immunosuppresseur de la famille des « limus » et les comprimés comprenant lesdites formulations excipients/ macrolide immunosuppresseur de la famille des « limus ».

La présente invention concerne également le procédé de préparation des formulations excipients/ macrolide immunosuppresseur de la famille des « limus ».

Les macrolides immunosuppresseur de la famille des « limus » sont représentés par le tacrolimus, le sirolimus et leurs analogues tels que le temsirolimus.

Le tacrolimus, aussi connu sous le nom de FK-506 ou de Fujimycine, est synthétisé par une bactérie, *Streptomyces tsukubaensis.* Ce produit est représenté par la formule (I) et a pour nom chimique le - 5,6,8,11,12,13,14,15,16,17,18,19,24,25,26,26 a-hexadecahydro-5, 19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-c] [1,4] oxaazacyclotricosine-1,7,20,21 (4H,23H)-tetrone.

Le tacrolimus commercialisé sous le nom de Prograf^{®} se présente sous forme de dispersion solide obtenue par co-précipitation en présence de solvants organiques.

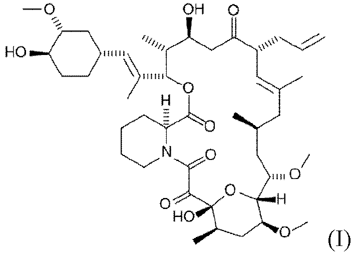

Le sirolimus ou rapamycine est un lactame-macrolide immunosuppresseur qui est produit par *Streptomyces hygroscopicus.* Le brevet US 3 929 992 décrit la préparation du sirolimus. Ce produit est représenté par la formule (II) et a pour nom chimique le (3*S*,6*R*,7*E*,9*R*,10*R*,12*R*,14*S*,15*E*,17*E*,19*E*,21*S*,23*S*,26*R*,27*R*,34a*S*)-9,10,12,13,14,21,22,23,24,25,26,27,32,33,34,34a-hexadecahydro-9,27-dihydroxy-3-[(1*R*)-2-[(1*S*,3*R*,4*R*)-4-hydroxy-3-methoxycyclohexyl]- 1-methylethyl]-10,21-dimethoxy-6,8,12,14,20,26-hexamethyl-23,27-epoxy-3*H-*pyrido[2,1-c][1,4]-oxaazacyclo hentriacontine - 1,5,11,28,29 (4*H*,6*H*,31*H*) - pentone.

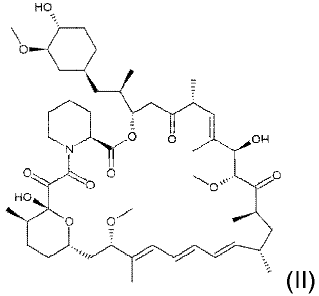

Le sirolimus utilisé dans la prévention de rejet de greffe rénale a été commercialisé sous le nom RAPAMUNE^{®}, spécialité se présentant sous la forme de comprimés enrobés (1 mg ou 2 mg) dans lequel le sirolimus nanodispersé selon la technologie Nanocrystal® en présence de poloxamer 188 se trouve dans l'une des couches de pelliculage du comprimé. La technologie Nanocrystal® est décrite comme une technologie de broyage en voie humide conduisant à l'obtention d'une dispersion de nanoparticules dans un milieu aqueux contenant des agents de stabilisation. Il est donc nécessaire de convertir cette dispersion en forme sèche pour produire une forme pharmaceutique commerciale stable, ce qui peut représenter un travail de développement complexe et présenter des risques de dégradation ou de perte de principe actif lors des étapes de conversion en forme sèche.

L'utilisation par voie orale des macrolides immunosuppresseurs de la famille des « limus » est limitée par leur biodisponibilité très faible et variable. Ces composés sont fortement insolubles dans les milieux biologiques, ce qui rend difficile leur formulation.

La demande de brevet WO2007091109 décrit une composition sous forme de granulés à base de tacrolimus amorphe préalablement dissous dans l'éthanol et comprenant au moins un excipient diluant et un excipient liant.

Le brevet EP0868911 concerne des compositions de sirolimus comprenant un noyau et un enrobage de sucre, ledit enrobage de sucre contenant du sirolimus, un ou plusieurs sucres et un ou plusieurs agents modificateurs de surface.

Le brevet EP0839028 concerne des compositions de rapamycine amorphe sous forme de dispersion solide dans un polymère hydrosoluble ou une cyclodextrine.

La demande de brevet EP1938800 concerne des nanodispersions de sirolimus comprenant un modificateur de surface et des particules de sirolimus de taille moyenne comprise entre 400 et 1200 nm.

En général, la nanonisation d'un principe actif très peu soluble dans l'eau permet d'améliorer sa solubilité apparente. Toutefois, plusieurs inconvénients résultent de cette diminution de taille. Les particules nanonisées ne sont pas stables et tendent à s'agglomérer, elles sont donc le plus souvent produites sous formes de dispersions dans un milieu aqueux contenants des agents de stabilisation. D'autre part, le broyage à des dimensions sub-microniques par des procédés mécaniques présente un risque élevé de changement de cristallinité et/ou de polymorphisme du principe actif avec un risque de production d'une proportion importante de forme amorphe. Or, la forme amorphe est moins stable que la forme cristalline et évoluera vers une forme cristalline au cours du temps avec un impact potentiel sur le comportement en dissolution et chez l'homme.

La demande de brevet WO2007/067566 décrit la préparation d'un dérivé de sirolimus sous forme cristalline à partir de matière brute telle qu'obtenue selon le procédé décrit dans le brevet US 5,985,325. La taille des particules des dérivés de sirolimus est supérieure à 30µm.

Cette demande de brevet décrit une méthode de cristallisation classique dans laquelle le macrolide est purifié après dissolution dans une solution d'acétate d'éthyle chauffée à 52-58°C, solution qui est ensuite filtrée. Un solvant de précipitation est ensuite ajouté.

Par ailleurs, il est précisé dans le texte de cette demande que la cristallinité du sirolimus ou de ses dérivés, contribue à la qualité générale du produit en particulier en termes de stabilité à la dégradation oxydative ; les formes amorphes ou partiellement cristallines étant soumises à une dégradation oxydative rapide.

Il est donc important de pouvoir préparer une forme cristalline du sirolimus et en particulier au moyen d'un procédé simplifié, ne mettant pas en oeuvre de solvant organique et sous forme de particules fines, de manière préférable de particules submicroniques.

La présente invention a donc pour objet une composition pharmaceutique pour administration orale caractérisée en ce qu'elle comprend une formulation excipient/produit d'un macrolide immunosuppresseur de la famille des « limus » contenant des particules d'au moins un excipient pharmaceutiquement acceptable sur lesquelles sont captées des particules fines cristallines d'un macrolide immunosuppresseur de la famille des « limus » selon l'invention.

Un autre objet de l'invention concerne une composition pharmaceutique pour administration orale à base d'un macrolide immunosuppresseur de la famille des « limus » comprenant :
(a) des particules d'au moins un excipient pharmaceutiquement acceptable,
(a) sur lesquelles sont captées des particules fines cristallines d'un macrolide immunosuppresseur de la famille des « limus » selon l'invention, ladite composition pharmaceutique est sous forme de
gélule ou de comprimé.

Un autre objet de l'invention concerne un procédé de préparation d'une formulation excipient/produit de l'invention.

Un autre objet de l'invention concerne un procédé de préparation d'une composition pharmaceutique pour administration orale selon l'invention.

### Définitions au sens du présent exposé de l'invention :

Par « particules fines » d'un macrolide immunosuppresseur de la famille des « limus »», on entend des particules d'une taille moyenne inférieure à quelques microns, de préférence d'une taille submicronique. Les particules fines présentent de préférence un diamètre volumique médian (dv(0,5)) inférieur à 2 µm, de manière encore préférée inférieur à 1 µm, de manière particulièrement préférée inférieur à 500 nm. 90% des particules fines présentent de préférence un diamètre volumique (dv(0,9)) inférieur à 5 µm, de manière encore préférée inférieur à 2 µm.

Les termes « forme cristalline » ou « particule cristalline » désignent au sens de la présente description, un produit sous forme polymorphique stable.

Par « forme polymorphique », on entend une structure organisée n'impliquant que des molécules de produit, possédant une empreinte cristalline caractéristique.

Par « exempt de solvant », selon la présente invention, on entend non préparé sous forme de dispersion, dans par exemple l'eau, l'éthanol, l'alcool isopropylique ou leurs mélanges.

Par « exempt d'agent modificateur de surface », selon la présente invention, on entend exempt d'agent utilisé pour disperser le produit dans un solvant et augmenter la mouillabilité du produit, comme par exemple la gélatine, la caséine, la lécithine, la gomme d'acacia, l'acide stéarique, le stéarate de calcium, la carboxymethylcellulose et ses dérivés, la méthylcellulose, l'hydroxyéthylcellulose, l'hydroxypropylcellulose, l'hydroxypropylméthycellulose phthalate.

Par « produit », selon la présente invention, on entend un macrolide immunosuppresseur de la famille des « limus ».

Par « formulation excipient/produit», selon la présente invention, on entend des particules fines cristallines d'un macrolide immunosuppresseur de la famille des « limus » captées sur des particules d'au moins un excipient pharmaceutiquement acceptable. Les particules d'excipients peuvent être des poudres directement compressibles ou des granules d'une granulométrie comprise entre 75 et 600 µm.

Par « captées », selon la présente invention, on entend déposées uniformément à la surface et/ou piégées à l'intérieur des pores des particules d'excipient.

Par « fluide à pression supercritique », on entend soit un fluide supercritique, c'est-à-dire un fluide porté à une pression et une température respectivement supérieures à sa pression et à sa température critique dans le cas d'un corps pur, soit par un point représentatif (pression, température) situé au-delà de l'enveloppe des points critiques représentés sur un diagramme (pression, température) dans le cas d'un mélange ; soit un liquide dit "sub-critique", c'est-à-dire un liquide qui se trouve dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange. Il est à noter que les propriétés physico-chimiques du dioxyde de carbone ainsi que ses paramètres critiques (pression critique : 7,4 MPa et température critique : 31 °C) en font le fluide préféré dans de nombreuses applications, d'autant qu'il ne présente pas de toxicité et est disponible à très bas prix en très grande quantité.

### DESCRIPTION DES FIGURES

La figure 1.1 est un schéma du fonctionnement général de l'installation de production de particules cristallines fines selon l'invention.
La figure 1.2 est un schéma montrant les résultats d'analyse enthalpique différentielle à balayage (DSC) pour caractérisation de l'état solide du sirolimus.
La figure 2.1 est un thermogramme de formulations sirolimus/mannitol (essai 2-1) et du mélange physique correspondant.
La figure 2-2 est un thermogramme de formulations sirolimus/lactose (essai 2-2) et du mélange physique correspondant.

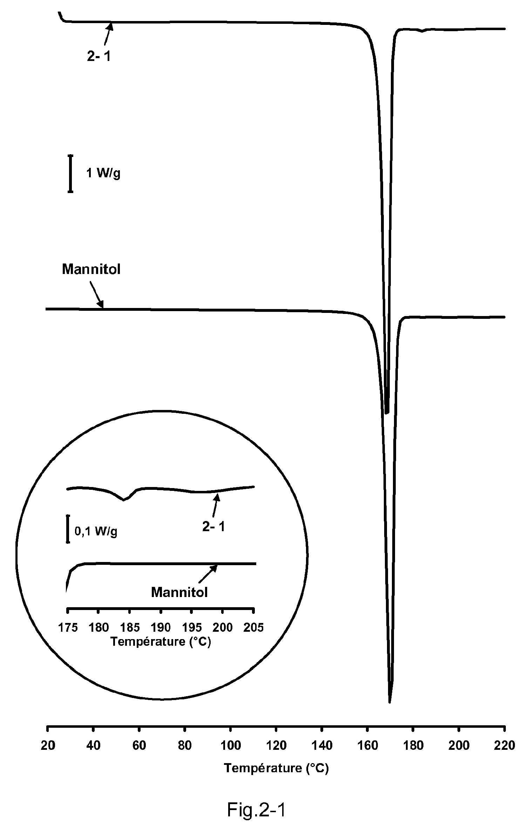

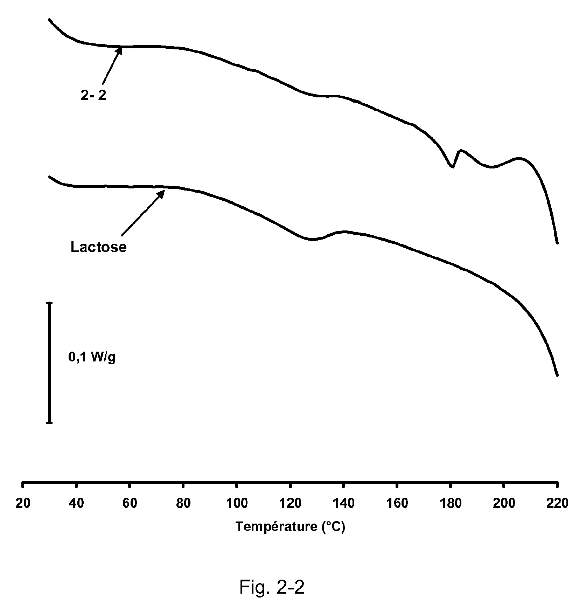

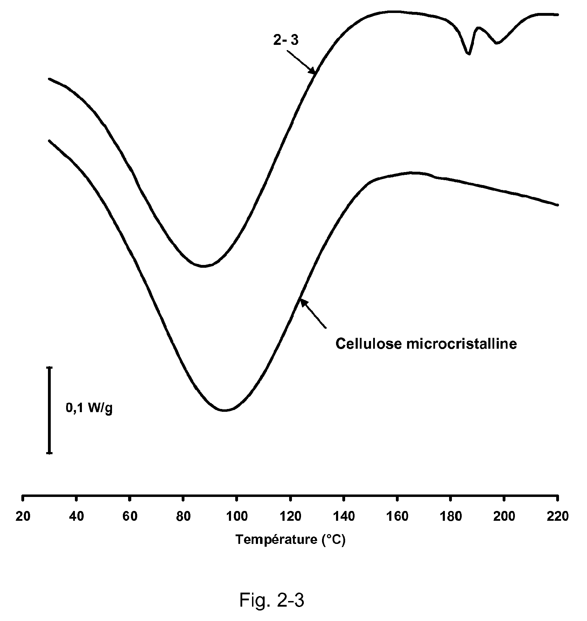

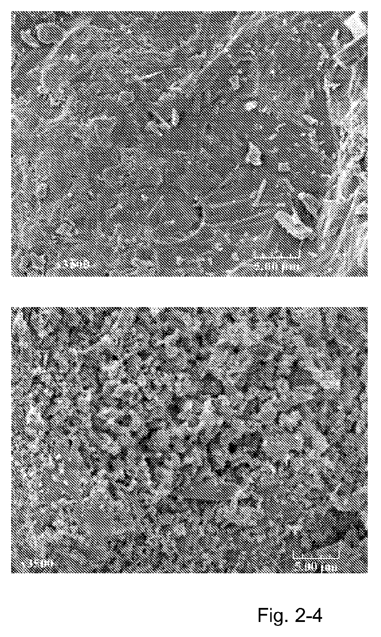

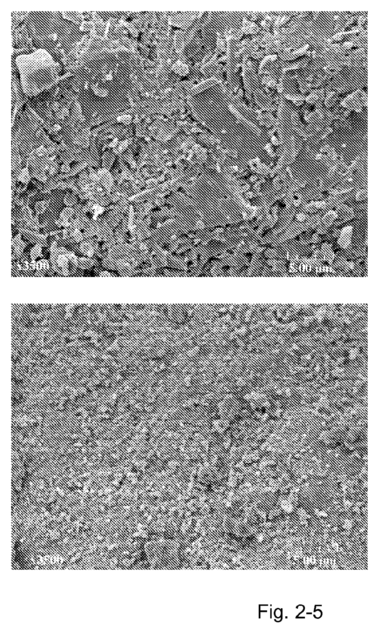

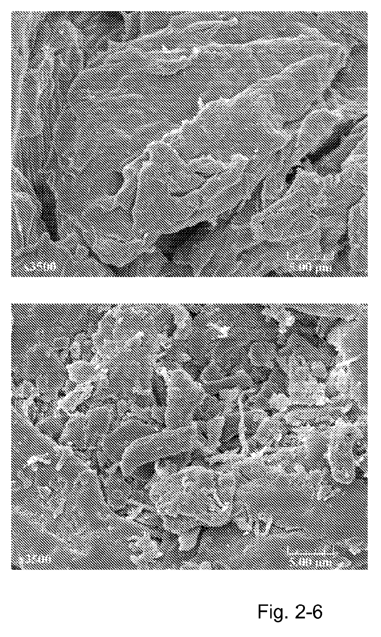

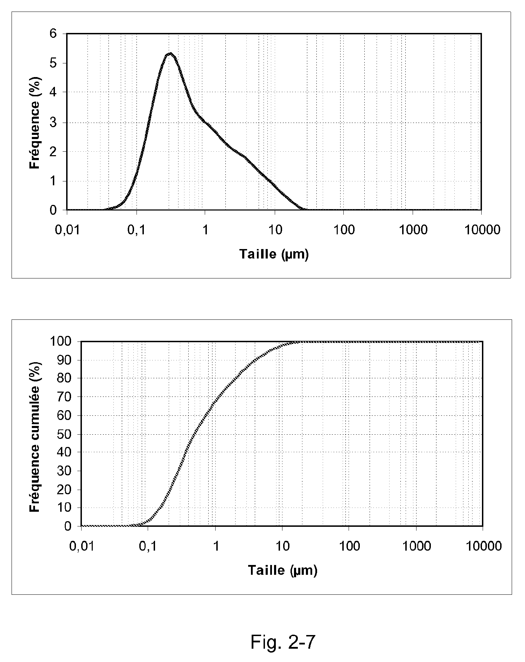

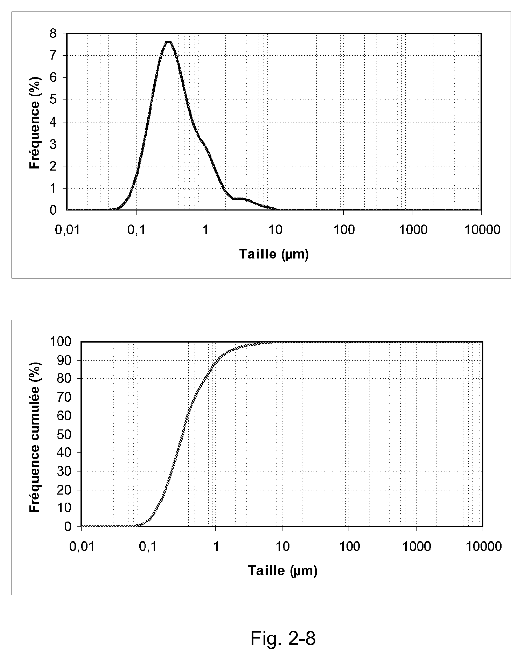

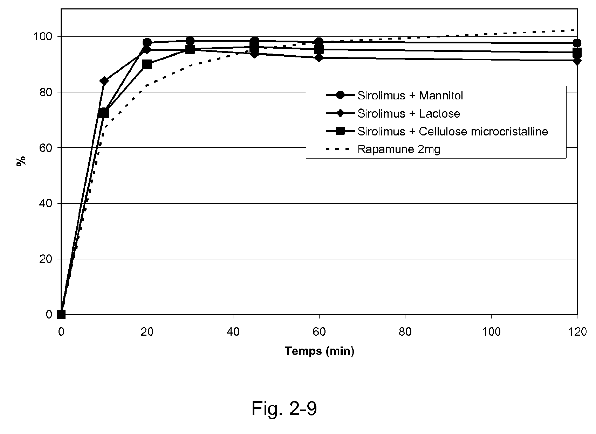

La figure 2-3 est un thermogramme de formulations sirolimus/cellulose (essai 2-3) microcristalline et du mélange physique correspondant.
La figure 2-4 est un schéma comparant des observations au microscope électronique à balayage pour le mannitol pur (haut) et la formulation sirolimus/mannitol (Essai 2-1) (bas).
La figure 2-5 est un schéma comparant des observations au microscope électronique à balayage pour le lactose pur (haut) et la formulation sirolimus/lactose (Essai 2-2) (bas).
La figure 2-6 est un schéma comparant des observations au microscope électronique à balayage pour la cellulose microcristalline pure (haut) et la formulation sirolimus/cellulose microcristalline (Essai 2-3) (bas).
La figure 2-7 est un schéma montrant la distribution de taille de particules pour la formulation sirolimus/mannitol (essai 2-1).
La figure 2-8 est un schéma montrant la distribution de taille de particules pour la formulation sirolimus/lactose (essai 2-2).
La figure 2-9 est un schéma comparant des courbes de dissolution in vitro des formulations sirolimus/excipient conditionnées en gélules vs comprimé de Rapamune^{®} 2 mg.

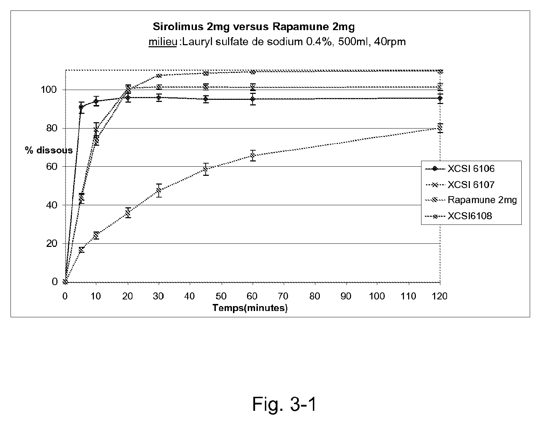

La figure 3-1 est un schéma comparant des courbes de dissolution in vitro des formulations sirolimus/excipient conditionnées sous forme de comprimé vs comprimé de Rapamune^{®} 2 mg.

### DESCRIPTION DETAILLEE DE L'INVENTION

Un premier objet de l'invention concerne une composition pharmaceutique pour administration orale caractérisée en ce qu'elle comprend une formulation excipient/produit d'un macrolide immunosuppresseur de la famille des « limus » contenant des particules d'au moins un excipient pharmaceutiquement acceptable sur lesquelles sont captées des particules fines cristallines d'un macrolide immunosuppresseur de la famille des « limus » selon l'invention.

Les particules d'excipients pharmaceutiquement acceptables peuvent être de toute nature chimique et préférentiellement formées d'un sucre, d'un polyol, d'amidon, d'un dérivé cellulosique, d'aluminosilicate de magnésium ou leurs mélanges.

Les particules d'excipients ont une granulométrie comprise entre 75 et 600 microns, de préférence entre 300 et 600 microns.

Les excipients pharmaceutiquement acceptables sont avantageusement choisis parmi le lactose (Pharmatose DCL21), la cellulose microcristalline (Avicel PH 200) et le mannitol (Pearlitol 200 SD).

Le taux de charge du macrolide immunosuppresseur de la famille des « limus » sur les particules d'excipients pharmaceutiquement acceptables est compris entre 0,5 et 10%, et préférentiellement entre 1 et 4%.

Un second objet de l'invention concerne une composition pharmaceutique pour administration orale à base d'un macrolide immunosuppresseur de la famille des « limus » comprenant :
a des particules d'au moins un excipient pharmaceutiquement acceptable,
b sur lesquelles sont captées des particules fines cristallines d'un macrolide immunosuppresseur de la famille des « limus » selon l'invention.

Une composition pharmaceutique selon l'invention peut se présenter sous forme de gélule ou de comprimé.

Selon un premier mode de réalisation de l'invention, une composition pharmaceutique selon l'invention est constituée de la formulation excipient/produit selon l'invention conditionnée sous forme de gélule.

Selon un second mode de réalisation de l'invention, une composition pharmaceutique selon l'invention est un comprimé comprenant uniquement la formulation excipient/produit selon l'invention. Avantageusement, les excipients pharmaceutiquement acceptables utilisés pour préparer la formulation excipient/produit selon l'invention sont alors préférentiellement choisis parmi les excipients connus de l'homme du métier pour présenter de bonnes propriétés de compression.

Les comprimés de l'invention peuvent comprendre optionnellement un ou plusieurs autres excipients choisi(s) dans le groupe constitué des agents diluants, des agents liants, des agents anti-statiques, des agents lubrifiants, des agents perméabilisants, des agents modificateurs de pH, des agents anti-oxydants, des édulcorants, des arômes et de leurs mélanges. Avantageusement, un comprimé de l'invention est constitué de la formulation excipient/produit selon l'invention et d'un ou plusieurs excipients choisi(s) dans le groupe constitué des agents diluants, des agents liants, des agents anti-statiques, des agents lubrifiants, des agents perméabilisants, des agents modificateurs de pH, des agents anti-oxydants, des édulcorants, des arômes et de leurs mélanges.

La proportion d'autres excipients par rapport à la formulation excipient/produit de l'invention dans le comprimé de l'invention peut être variable.

Avantageusement, le mélange d'excipients comprend :
- un ou des agents diluants permettant de garantir une masse de comprimé en adéquation avec les contraintes de fabrication. Tous les excipients pharmaceutiquement acceptables et décrits comme diluants peuvent être utilisés parmi lesquels les dérivés de la cellulose et le lactose,
- un ou des agents liants permettant d'obtenir des comprimés de propriétés physiques (dureté, friabilité) adaptées. Tous les excipients pharmaceutiquement acceptables et décrits comme liants peuvent être utilisés parmi lesquels les celluloses, les phosphates de calcium et le lactose,
- des agents anti-statiques, lubrifiants ou facilitateurs d'écoulement peuvent être ajoutés pour améliorer les propriétés du mélange à comprimés. Parmi ceux-ci les dérivés de la silice, le talc, le stéarate de magnésium, le stéarylfumarate de sodium,
- des agents modificateurs de pH permettant de créer un micro-pH autour de la molécule. Parmi ces agents, peuvent être considérés des agents basiques comme le bicarbonate de sodium ou le carbonate de calcium, ou des acides comme l'acide citrique ou l'acide fumarique,
- des colorants, édulcorants, aromatisants peuvent également être ajoutés en fonction des besoins de la formulation.
- des antioxydants.

Une composition pharmaceutique de l'invention sous forme de comprimé présente de préférence un taux de dissolution supérieur à 60%, de manière particulièrement préférée supérieur à 70% en 10 min.

Une composition pharmaceutique de l'invention sous forme de comprimé présente de préférence un taux de dissolution supérieur à 80%, de manière particulièrement préférée supérieur à 90% en 20 min.

Une composition pharmaceutique de l'invention sous forme de comprimé présente de préférence un taux de dissolution supérieur à 90%, de manière particulièrement préférée supérieur à 95% en 30 min.

Ces taux de dissolution s'entendent de préférence dans un milieu de dissolution composé de 0,4% de sodium dodecyl sulfate (SDS) dans l'eau conformément à la Pharmacopée européenne.

Une composition pharmaceutique de l'invention comprend avantageusement 1 ou 2 mg de produit.

La présente invention concerne également un procédé de préparation des fines particules cristallines de produit de l'invention et des formulations excipient/produit de l'invention.

Les fines particules cristallines de produit de l'invention et les formulations excipient/produit de l'invention sont obtenues dans des conditions particulièrement avantageuses au moyen d'un procédé utilisant un fluide à pression supercritique. Parmi les procédés de production de particules fines utilisant un fluide supercritique, on connaît par exemple le procédé connu sous l'acronyme RESS, pour « rapid expansion of supercritical solutions » décrit dans les documents US 4,582,731 et WO01/43853.

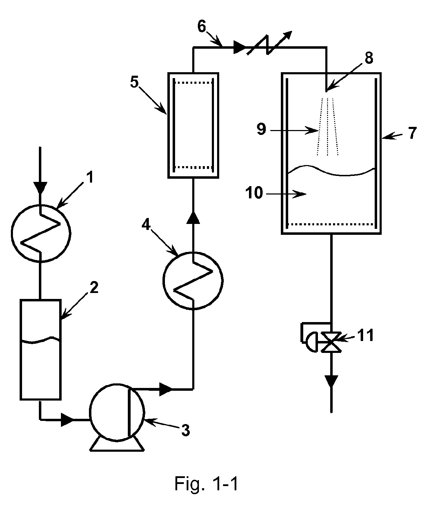

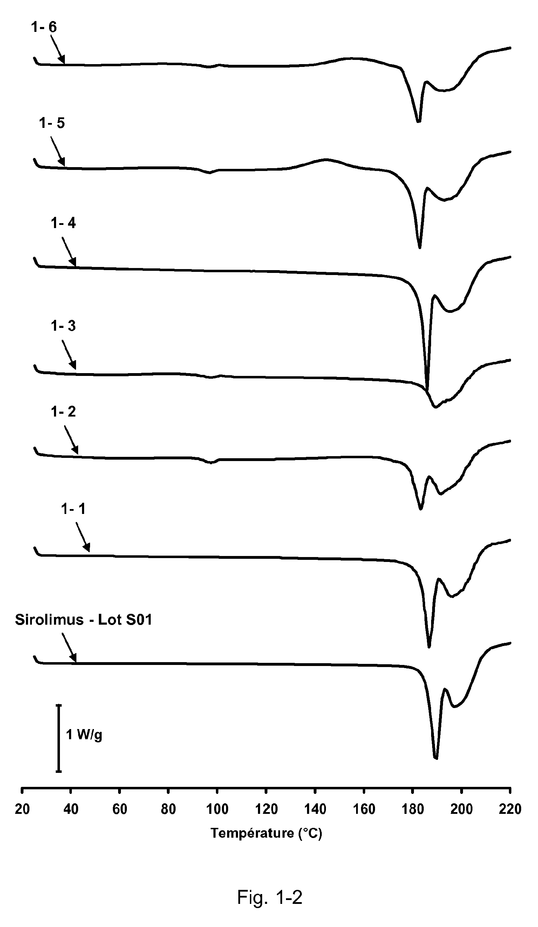

On décrira tout d'abord le fonctionnement général de l'installation selon la Figure 1-1 puis le fonctionnement spécifique de cette installation pour la préparation d'une forme cristalline d'un composé de type « limus », ainsi que les moyens mis en oeuvre pour obtenir une formulation excipient/sirolimus composée de particules d'excipients sur lesquelles sont captées des particules cristallines d'un composé de type « limus ».

Suivant la mise en oeuvre la plus courante de la technique dite RESS, le produit à microniser ou nanoniser est placé dans une cellule d'extraction (tube fermé aux extrémités par des frittés en acier inoxydable pour éviter l'entraînement du produit par le fluide). Cette cellule est placée à l'intérieur d'un autoclave d'extraction (5) chauffé à la température d'extraction choisie. Le fluide est pompé (3) depuis le stockage (2) et traverse un échangeur chaud (4) régulé à la température d'extraction désirée avant d'entrer dans l'autoclave d'extraction. La solution de produit solubilisé dans le fluide à pression supercritique sortant de l'autoclave d'extraction est envoyée vers une enceinte de pulvérisation (7) dans laquelle la solution est dépressurisée brutalement à une température et une pression donnée à travers une buse (8). Un dispositif de chauffage (6) situé immédiatement en amont de l'autoclave de pulvérisation permet de régler la température de pré-expansion du fluide. La chute de pression à travers la buse induit une chute brutale de la solubilité du produit dans le fluide, conduisant à la formation de particules fines au sein de l'enceinte de pulvérisation. Cette enceinte de pulvérisation est opérée le plus souvent à pression atmosphérique.

Dans une mise en oeuvre avantageuse, l'enceinte de pulvérisation contient un panier de collecte dont le fond est fermé par un filtre afin d'éviter l'entraînement des fines particules formées par le fluide. Le fluide est ensuite évacué dans l'atmosphère ou éventuellement recomprimé et recyclé. Le procédé RESS offre donc la possibilité de produire en une étape, en l'absence de solvant et/ou d'agents tensio-actifs, des particules fines sèches, d'un produit.

Dans le cadre de la présente invention, il est apparu de manière surprenante que l'utilisation d'une pression sensiblement supérieure à la pression atmosphérique dans l'enceinte de pulvérisation et réglée à l'aide de la vanne de régulation de pression amont (11) permet, indépendamment de la température de pré-expansion réglée par le dispositif de chauffage (6), d'obtenir de fines particules cristallines d'un macrolide immunosuppresseur de la famille des « limus » avec un état solide proche de celui du produit cristallin de départ, sans ou avec peu de phase amorphe, ce qui devrait permettre d'assurer la stabilité à long terme de l'état solide du macrolide immunosuppresseur de la famille des « limus » au sein des particules puisque l'on obtient le principe actif sous une forme cristalline stable sans risque de recristallisation d'une phase amorphe, mais aussi sa stabilité chimique dans la mesure où la présence de phase amorphe dans des poudres du macrolide immunosuppresseur de la famille des « limus » est connue comme un facteur diminuant la stabilité de ce type de principe actif.

Un autre objet de l'invention concerne donc un procédé de préparation de fines particules cristallines d'un macrolide immunosuppresseur de la famille des « limus »comprenant les étapes suivantes :
- solubilisation d'un macrolide immunosuppresseur de la famille des « limus » dans un fluide à pression supercritique dans un autoclave d'extraction (5)
- dépressurisation brutale de la solution obtenue à l'étape précédente à travers une buse (8) dans une enceinte de pulvérisation (7), la pression dans l'enceinte de pulvérisation étant sensiblement supérieure à la pression atmosphérique.

De préférence, le fluide utilisé est le CO₂, porté à une pression supérieure à sa pression critique (7,4 MPa), entre 10 et 100 MPa, et de préférence entre 10 et 40 MPa, et à une température supérieure à sa température critique (31°C), de préférence entre 35 et 80°C, dans l'autoclave d'extraction.

De préférence, la précipitation du macrolide immunosuppresseur de la famille des « limus » est obtenue par détente de la solution dudit macrolide dans le CO₂ supercritique porté à une température de pré-expansion de 60 à 140°C, et de préférence entre 60 et 100°C.

De préférence, la pression dans l'enceinte de pulvérisation (7) est comprise entre 1 et 7 MPa, et de préférence entre1 et 4 MPa.

Un autre objet de l'invention concerne un procédé de préparation de la formulation excipient/produit de l'invention.

La technique RESS, en particulier le procédé de préparation de fines particules solides de l'invention décrit ci-dessus, est alors mise en oeuvre avec une étape de capture des particules formées sur un lit d'excipient (10) mis en place dans l'enceinte de pulvérisation (7). Cette mise en oeuvre particulière, décrite dans le brevet EP 1 239 938 permet de capturer de manière très efficace les particules formées au cours du procédé. En outre, cette mise en oeuvre permet d'améliorer de manière très significative la qualité d'usage des fines particules du macrolide immunosuppresseur de la famille des « limus » par rapport à la mise en oeuvre classique de la technique RESS, les fines particules étant déposées sur des particules d'au moins un excipient pharmaceutiquement acceptable tel que décrit ci-dessus.

Dans un mode de mise en oeuvre préféré du procédé de préparation de la formulation excipient/produit de l'invention :
- le fluide utilisé est le CO₂, porté à une pression supérieure à sa pression critique (7,4 MPa), entre 10 et 100 MPa, et de préférence entre 10 et 40 MPa, et à une température supérieure à sa température critique (31°C), de préférence entre 35 et 80°C, dans l'autoclave d'extraction,
- la précipitation du macrolide immunosuppresseur de la famille des « limus » est obtenue par détente de la solution de dit macrolide dans le CO₂ supercritique portée à une température de pré-expansion de 60 à 140°C, et de préférence entre 60 et 100°C,
- la pression dans l'enceinte de pulvérisation (7) est comprise entre 1 et 7 MPa, et de préférence entre1 et 4 MPa,
- on capture des particules fines cristallines du macrolide immunosuppresseur de la famille des « limus » formées sur un lit d'au moins un excipient pharmaceutiquement acceptable (10) mis en place dans l'enceinte de pulvérisation (7).

La présent invention a aussi pour objet une composition pharmaceutique pour son utilisation en tant que traitement immunosuppresseur à titre préventif et/ou curatif, en particulier pour traiter et/ou prévenir un rejet après une greffe d'organe, par exemple de rein.

Une composition pharmaceutique selon l'invention peut être utilisée dans une méthode de traitement d'un patient ayant reçu une greffe d'organe, par exemple de rein, en une quantité suffisante pour traiter et/ou prévenir le rejet de l'organe greffé.

La présente invention est illustrée plus en détail à l'aide des exemples descriptifs ci après.

### EXEMPLES

### Exemple 1 : Production de particules de sirolimus pures par le procédé RESS

Le procédé RESS est mis en oeuvre pour la production de particules de sirolimus pur, sans excipient dans l'enceinte de pulvérisation, avec un équipement de taille laboratoire correspondant au schéma décrit sur la Figure 1-1.

0,5 g de sirolimus (Lot S01) mélangé à environ 6 g de billes de verre de 1 mm de diamètre est disposé dans une cellule d'extraction de 10 mL. Ladite cellule est placée à l'intérieur de l'autoclave d'extraction (5) chauffé à une température de 60°C. Après une étape de mise en régime, le dioxyde de carbone à l'état supercritique, à une pression de 40 MPa, une température de 60°C et un débit de 0,3 kg/h, percole à travers la cellule d'extraction pour extraire le sirolimus. La solution de sirolimus dans le dioxyde de carbone supercritique est détendue à travers une buse de pulvérisation (8) constituée d'un capillaire PEEK de diamètre interne 63 µm et de longueur adaptée à la pression et au débit de travail. La température de pré-expansion est réglée via un dispositif de chauffage (6) situé immédiatement en amont de la buse. L'enceinte de pulvérisation (7), d'un volume intérieur utile de 65 mL, est chauffée à 60°C. La pression au sein de l'enceinte de pulvérisation est réglée par une vanne de régulation de la pression amont (11). Après 5 heures de mise en oeuvre, la pompe de CO₂ est arrêtée et on diminue graduellement la pression dans l'enceinte de pulvérisation jusqu'à pression atmosphérique en environ 30 minutes avant collecte du produit.

Deux séries de trois essais ont été menées. Chaque série correspond à une température de pré-expansion de la solution supercritique.

Plus précisément, la première série d'essais est opérée avec une température de pré-expansion de 60°C La détente opérée à travers la buse est très brève et est généralement supposée isenthalpe. Par conséquent, l'homme de l'art peut déterminer qu'une détente opérée dans ces conditions conduit à la présence d'un mélange de CO₂ liquide et de CO₂ gazeux immédiatement en aval de la buse. La seconde série d'essais est opérée avec une température de pré-expansion de 140°C, soit environ la température minimale de pré-expansion requise pour que le CO₂ soit intégralement à l'état gazeux en aval de la buse de pulvérisation.

Pour chaque série, des essais sont opérés avec des pressions dans l'enceinte de pulvérisation de 3, 1 et 0,1 MPa. Le Tableau 1-1 récapitule les paramètres utilisés pour les six essais.

**Tableau 1-1 : Conditions opératoires pour les essais 1-1 à 1-6**

| Numéro de l'essai | Température de pré-expansion de la solution supercritique (°C) | Pression dans l'enceinte de pulvérisation (MPa) |
|---|---|---|
| 1-1 | 60 | 3 |
| 1-2 | 60 | 1 |
| 1-3 | 60 | 0,1 |
| 1-4 | 140 | 3 |
| 1-5 | 140 | 1 |
| 1-6 | 140 | 0,1 |

Pour chaque essai, une masse d'environ 150 mg de sirolimus a été extraite et un échantillon d'environ 100 mg de particules de sirolimus a été collecté dans l'enceinte de pulvérisation.

### Résultats

Les particules fines de sirolimus obtenues par le procédé RESS ont été caractérisées par analyse enthalpique différentielle à balayage (DSC) pour caractérisation de l'état solide du sirolimus au sein des particules et par chromatographie en phase liquide à haute performance (CLHP) pour quantification du titre et du profil de substances apparentées.

Les résultats d'analyse calorimétrique différentielle à balayage (DSC) (Figure 1-2) montrent que, pour les deux séries d'essais, une pression dans l'enceinte de pulvérisation de 3 MPa permet de produire une matière cristalline avec un état solide similaire à celui de la matière première alors que des pressions de pulvérisation de 1 et 0,1 MPa conduisent à l'obtention de particules de sirolimus de moindre taux de cristallinité. En effet, les courbes sur la Figure 1-2 mettent en évidence une diminution sensible de l'enthalpie globale du pic endothermique principal correspondant à la fusion du sirolimus entre 185 et 195°C avec la pression de pulvérisation, quelle que soit la température de pré-expansion. Les particules de sirolimus obtenues à une pression de pulvérisation de 1 ou 0,1 MPa sont très probablement constituées du mélange d'une phase cristallisée et d'une phase amorphe.

L'analyse par chromatographie en phase liquide à haute performance indique une bonne stabilité du sirolimus au cours du procédé pour les essais opérés avec une température de pré-expansion de 60°C : titre et profil de substances apparentées sont similaires à ceux du produit de départ pour l'ensemble des échantillons. En revanche, cette même analyse révèle que l'aire relative d'un pic chromatographique (Temps de rétention relatif = 1,56) pour les essais menés avec une température de pré-expansion de 140°C est plus importante que pour le produit de départ. Il est de plus à noter que ce pic chromatographique n'est pas observé pour les particules de sirolimus produites avec une température de pré-expansion de 60°C.

Par conséquent, il apparaît que des conditions opératoires particulières (température de pré-expansion de 60°C et pression dans l'enceinte de pulvérisation de 3 MPa) permettent à la fois de conserver la cristallinité du sirolimus et d'assurer la stabilité du produit par rapport au produit de départ au cours du procédé. En revanche, ces conditions opératoires conduisent à de faibles rendements de collecte et à des poudres de manipulation peu aisée.

### Exemple 2 : Production de formulations excipient/produit (sirolimus) par le procédé RESS

Le procédé RESS est mis en oeuvre pour la production de particules de sirolimus déposées sur un excipient préalablement déposé dans l'enceinte de pulvérisation, avec un équipement de taille pilote correspondant au schéma décrit sur la Figure 1-1.

50 g d'excipient sous forme de poudre (10) est initialement placé dans le panier de collecte situé dans l'enceinte de pulvérisation. 1,25 g de sirolimus (Lot S02) mélangé à environ 20 g de billes de verre de 1 mm de diamètre est disposé dans une cellule d'extraction de 20 mL. Ladite cellule est placée à l'intérieur de l'autoclave d'extraction (5) chauffé à une température de 60°C. Après une étape de mise en régime, le dioxyde de carbone à l'état supercritique, à une pression de 33 MPa, une température de 60°C et un débit de 2,0 kg/h, percole à travers la cellule d'extraction pour extraire le sirolimus. La solution de sirolimus dans le dioxyde de carbone supercritique est détendue à travers une buse de pulvérisation (8) constituée d'un capillaire PEEK de diamètre interne 127 µm et de longueur adaptée à la pression et au débit de travail. L'enceinte de pulvérisation (7), d'un volume intérieur utile de 300 mL, est chauffée à 60°C. Cette même enceinte est équipée d'un agitateur à entraînement magnétique sur l'arbre duquel est adapté une pale de type ancre marine, permettant, en cas de besoin, d'uniformiser la répartition de l'excipient dans le panier de collecte. Le procédé est opéré avec une température de pré-expansion de 60°C et une pression dans l'enceinte de pulvérisation de 3 MPa soient les conditions optimisées à partir de l'exemple 1 en termes de contrôle de l'état solide du sirolimus et de stabilité chimique du produit au cours du procédé. Après 5 heures de mise en oeuvre, la pompe de CO₂ est arrêtée et on diminue graduellement la pression dans l'enceinte de pulvérisation jusqu'à pression atmosphérique en environ 30 minutes avant collecte du produit.

Des essais ont été opérés avec trois excipients différents comme indiqué dans le Tableau 2-1.

**Tableau 2-1 : Excipients utilisés pour les essais 2-1 à 2-3**

| Numéro de l'essai | Type d'excipient | Nom commercial de l'excipient utilisé |
|---|---|---|
| 2-1 | Mannitol | Pearlitol 200 SD |
| 2-2 | Lactose | Pharmatose DCL21 |
| 2-3 | Cellulose microcristalline | Avicel PH 200 |

Dans les conditions expérimentales choisies, environ 1,20 g de sirolimus a été solubilisé et pulvérisé sur environ 50 g d'excipient au cours de chaque essai soit un taux de charge théorique en sirolimus pour chaque formulation d'environ 2,3 % en masse. Pour les trois excipients testés, la formulation finale collectée dans le panier de l'enceinte de pulvérisation est physiquement homogène (pas d'agglomérats) et présente le même aspect que les particules d'excipient initiales.

### Résultats

Pour chaque essai, l'échantillon collecté a été caractérisé par :
- analyse enthalpique différentielle à balayage (DSC) pour caractérisation de l'état solide du sirolimus au sein de la formulation,
- chromatographie en phase liquide à haute performance (CLHP) pour mesure des taux de charge réel et vérification du profil de substances apparentées,
- microscopie électronique à balayage (MEB) pour observation de la surface des particules d'excipient telles que reçues et des formulations sirolimus/excipient,
- granulométrie laser par diffraction de lumière après dispersion des formulations dans un véhicule aqueux et dissolution de l'excipient pour mesure de la taille des particules de sirolimus dans la formulation, uniquement pour les formulations sur mannitol et lactose, la cellulose ne pouvant être solubilisée dans un tel milieu aqueux,
- test de dissolution *in vitro* pour comparaison par rapport à une formulation de référence.

Les Figures 2-1 à 2-3 présentent pour les trois essais la comparaison des thermogrammes de l'excipient pur et de la formulation sirolimus/excipient. Pour chacune des trois formulations sirolimus/excipient, le thermogramme de la formulation est très proche de celui de l'excipient seul et ne met pas en évidence d'anomalie particulière. Dans chacun des cas, deux pics endothermiques caractéristiques du sirolimus à environ 183 et 195°C sont identifiables et indicatifs de la présence de sirolimus cristallin. Un pic endothermique majoritaire à 166°C correspondant à celui observé pour l'excipient pur apparaît dans le cas du mannitol.

Les résultats d'analyse par CLHP mettent en évidence une uniformité de taux de charge élevée des formulations (voir Tableau 2-2). Par ailleurs, les taux de charge mesurés sont très proches du taux de charge théorique de 2,3% en masse, indiquant que la mise en oeuvre du procédé RESS avec capture sur lit d'excipient permet de collecter efficacement les particules de sirolimus. Par ailleurs, la comparaison des profils de substances apparentées entre les formulations sirolimus/excipient obtenues par le procédé RESS et les mélanges physiques sirolimus de départ/excipient correspondants met en évidence la stabilité du sirolimus déposé sur l'excipient par le procédé par rapport au sirolimus de départ.

**Tableau 2-2 : Résultats de mesure de taux de charge pour les essais 2-1 à 2-3**

| Référence de l'échantillon | Moyenne des taux de charge mesurés (%) | Ecart type | Coefficient de variation (%) |
|---|---|---|---|
| 2-1 | 2,07 | 0,04 | 1,9 |
| 2-2 | 2,00 | 0,05 | 2,3 |
| 2-3 | 2,41 | 0,01 | 0,4 |

Les Figures 2-4 à 2-6 présentent les images respectives obtenues en microscopie électronique à balayage pour les échantillons 2-1 à 2-3. Sauf dans le cas de la cellulose microcristalline (Figure 2-6), il apparaît très nettement que de très fines particules de sirolimus sont déposées uniformément à la surface des particules d'excipient. Dans le cas de la cellulose cristalline, la présence de très fines particules de sirolimus est difficile à observer et la surface relativement poreuse des particules d'excipient laisse supposer que des particules de sirolimus sont piégées à l'intérieur des anfractuosités des particules de cellulose.

Dans le cas des formulations déposées sur mannitol et sur lactose, la présence de très fines particules de sirolimus est confirmée par les mesures effectuées au granulomètre laser par diffraction de lumière, en milieu aqueux et après dissolution complète des particules d'excipient. Les Figures 2-7 et 2-8 présentent les courbes de distribution de taille de particules respectives pour la formulation sur mannitol et pour celle sur lactose. Comme indiqué dans le Tableau 2-3, les particules de sirolimus obtenues par le procédé de l'invention présentent des tailles très majoritairement submicroniques. Dans le cas de la formulation sur lactose, la mise en oeuvre du procédé est particulièrement efficace en termes de production de particules d'un diamètre volumique moyen dᵥ(0,5) de quelques centaines de nanomètres.

**Tableau 2-3 : Résultats de mesure de granulométrie laser (valeurs moyennes sur 3 prises d'essais)**

| Numéro de l'essai | Type d'excipient | Dᵥ[4,3] | dᵥ(0,1) | dᵥ(0,5) | dᵥ(0,9) |
|---|---|---|---|---|---|
| 2-1 | Mannitol | 1,742 | 0,176 | 0,567 | 4,681 |
| 2-2 | Lactose | 0,647 | 0,159 | 0,371 | 1,277 |

Dans le cadre des tests de dissolution *in vitro* opérés sur un appareil de type USP I, les formulations ont été conditionnées en gélules (LGA, taille 0, translucide, code 000020), en quantité équivalente à 2 mg de sirolimus, et analysées par comparaison avec des comprimés de Rapamune^{®} 2 mg. Le milieu de dissolution était constitué d'une solution de SDS à 0,4% à une température de 37°c et la vitesse de rotation du panier était fixée à 100 rpm. Les prélèvements ont été analysés par CLHP/UV après filtration. Les profils de dissolution *in vitro* présentés sur la Figure 2-9 montrent que les formulations mises en gélules ont une cinétique de dissolution similaire ou améliorée par rapport au produit de référence Rapamune^{®}.

Les conditions de dissolution présentées sur la Figure 2-9 sont 0.4% sodium laurylsulfate, 500mL, panier à 100rpm.

Les formulations sirolimus/excipient obtenues par le procédé de l'invention, excluant l'usage de tout solvant ou agent tensio-actif, simplement conditionnées en gélule permettent donc d'obtenir des performances de dissolution *in vitro* comparables à celle de la formulation de référence.

### Exemple 3 : Préparation et caractérisation de comprimés à partir des formulations de l'exemple 2

Des comprimés ont été préparés à partir de la formulation excipient/produit de l'exemple 2 et dosés à environ 2 mg de sirolimus.

Ces comprimés ont été fabriqués sur machine à comprimer rotative (SVIAC PR12) et les paramètres de compression ont été fixés pour obtenir des comprimés de masse de l'ordre de 337mg.

Les résultats compilés dans le Tableau 3.1 montrent la bonne reproductibilité des essais en termes de solubilisation du sirolimus et des rendements de collecte.

**Tableau 3-1 : Rendements de collecte et caractéristiques des comprimés**

| | Numéro de lot | **XCXC6106** | | **XCXC6107** | | **XCXC6108** | |
|---|---|---|---|---|---|---|---|
| | *Excipients* | Mannitol (Pearlitol 200 SD) | | Lactose (PharmatoseD CL21) | | Cellulose microcristallin e (Avicel PH200) | |
| | | % | mg/co mp. | % | mg/com p. | % | mg/co mp. |
| *Excipient s* | Essai 2.1 | 28,67 | 96,62 | - | - | - | - |
| | Essai 2.2 | - | - | 29,6 7 | 100,00 | - | - |
| | Essai 2.3 | - | - | - | - | 24,7 3 | 83,33 |
| *Diluant* | Mannitol | 71,13 | 239,71 | - | - | - | - |
| | Lactose | - | - | 70,1 3 | 236,33 | - | - |
| | Cellulose microcristalline | - | - | - | - | 75,0 7 | 253 |
| *Lubrifiant* | Stéarate de Magnésium | 0,20 | 0,68 | 0,20 | 0,68 | 0,20 | 0,67 |
| *TOTAL* | | 100,0 0 | 337,00 | 100, 00 | 337,00 | 100, 00 | 337,00 |
| *Titre Formulatio n* | Titre théorique (mg/g) | 20,0 | | 20,0 | | 20,0 | |
| | Titre réel (mg/g) | 20,7 (1,9%) | | 20,0 (CV=2,3%) | | 24,1 (CV=0,4%) | |
| *Caractéris -tiques physiques des comprimé* s | Masse moyenne (mg) (n=10) | 336,9 (CV=0,50%) | | 337,0 (CV=ND) | | 338,3 (CV=0,27%) | |
| | Epaisseur (mm) | 4,44 | | 4,23 | | 5,46 | |
| | Dureté (n=10) (N) | 188,0 (CV=9,0%) | | 131,0 (CV=6,6%) | | 173,8 (CV=3,6%) | |
| *Titre comprimé s* | Titre théorique (mg/g) | 2,00 | | 2,00 | | 2,00 | |
| | Titre réel (n=6) (mg/g) | 1,81 (CV=1,0%) | | 2,05 (CV=2,8%) | | 2,11 (CV=1,5%) | |

| | | | | | | | |
|---|---|---|---|---|---|---|---|
| CV : coefficient de variation exprimé en % | | | | | | | |

Les conditions de dissolution présentées sur la Figure 3-1 sont 500mL de milieu à 0,4% de laurylsulfate de sodium, panier à 40rpm

Les profils de dissolution *in vitro* montrent (Figure 3.1) que les formulations sous forme de comprimé ont une cinétique de dissolution améliorée par rapport au produit de référence, le Rapamune^{®}.

## Revendications

1. Composition pharmaceutique pour administration orale **caractérisée en ce qu'**elle comprend une formulation excipient/produit d'un macrolide immunosuppresseur de la famille des « limus » contenant des particules d'au moins un excipient pharmaceutiquement acceptable sur lesquelles sont captées des particules fines cristallines d'un macrolide immunosuppresseur de la famille des « limus ».

2. Composition pharmaceutique selon la revendication 1, dans laquelle la formulation excipient/produit est exempte d'agent modificateur de surface et de solvant.

3. Composition pharmaceutique selon la revendication 1 ou 2, dans laquelle le macrolide immunosuppresseur de la famille des « timus » est choisi dans le groupe comprenant le tacrolimus et le sirolimus.

4. Composition pharmaceutique selon l'une quelconque des revendications 1 à 3, dans laquelle l'excipient est choisi dans le groupe comprenant notamment un sucre, un polyol, l'amidon, un dérivé cellulosique, l'aluminosilicate de magnésium ou leurs mélanges.

5. Composition pharmaceutique selon la revendication 4, dans laquelle l'excipient est choisi dans le groupe comprenant notamment le lactose, la cellulose microcristalline, le mannitol et leurs mélanges.

6. Composition pharmaceutique selon la revendication 4 ou 5, dans laquelle le taux de charge du macrolide immunosuppresseur de la famille des « limus » sur des particules d'excipients pharmaceutiquement acceptables est compris entre 0,5 et 10%, et préférentiellement entre 1 et 4%.

7. Composition pharmaceutique selon l'une quelconque des revendications 1 à 6, sous forme de gélule ou comprimé.

8. Composition pharmaceutique selon la revendication 7, sous forme de comprimé constitué d'une formulation excipient/produit d'un macrolide immunosuppresseur de la famille des « limus » contenant des particules d'au moins un excipient pharmaceutiquement acceptable sur lesquelles sont captées des particules fines cristallines d'un macrolide immunosuppresseur de la famille des « limus » et optionnellement d'un ou plusieurs excipients choisi(s) dans le groupe constitué des agents diluants, des agents liants, des agents anti-statiques, des agents lubrifiants, des agents perméabilisants, des agents modificateurs de pH, des agents anti-oxydants, des édulcorants, des arômes et de leurs mélanges.

9. Composition pharmaceutique selon l'une quelconque des revendications 1 à 8, pour son utilisation en tant que traitement immunosuppresseur à titre préventif et/ou curatif, en particulier pour traiter et/ou prévenir un rejet après une greffe d'organe, par exemple de rein.

10. Procédé de préparation de fines particules cristallines de macrolide immunosuppresseur de la famille des « limus » comprenant les étapes suivantes :
- solubilisation d'un macrolide immunosuppresseur de la famille des « limus » dans du CO₂, porté à une pression supérieure à sa pression critique (7,4 MPa), entre 10 et 100 MPa, et de préférence entre 10 et 40 MPa, et à une température supérieure à sa température critique (31 °C), de préférence entre 35 et 80°C, dans un autoclave d'extraction (5)
- dépressurisation brutale de la solution obtenue à l'étape précédente à travers une buse (8) dans une enceinte de pulvérisation (7), la pression dans l'enceinte de pulvérisation (7) étant sensiblement supérieure à la pression atmosphérique, de préférence comprise entre 1 et 7 MPa, et de préférence entre 1 et 4 MPa.

11. Procédé de préparation d'une formulation excipient/produit comprenant les étapes suivantes :
- solubilisation d'un macrolide immunosuppresseur de la famille des « limus » dans du CO₂, porté à une pression supérieure à sa pression critique (7,4 MPa), entre 10 et 100 MPa, et de préférence entre 10 et 40 MPa, et à une température supérieure à sa température critique (31°C), de préférence entre 35 et 80°C, dans un autoclave d'extraction (5)
- dépressurisation brutale de la solution obtenue à l'étape précédente à travers une buse (8) dans une enceinte de pulvérisation (7), la pression dans l'enceinte de pulvérisation (7) étant sensiblement supérieure à la pression atmosphérique, de préférence comprise entre 1 et 7 MPa, et de préférence entre1 et 4 MPa
- capture des particules fines cristallines du macrolide immunosuppresseur de la famille des « limus » formées sur un lit d'excipient pharmaceutiquement acceptable (10) mis en place dans l'enceinte de pulvérisation (7).

## Patentansprüche

1. Pharmazeutische Zusammensetzung zur oralen Verabreichung, **dadurch gekennzeichnet, dass** sie eine Hilfsstoff-/Produkt-Formulierung eines immunsuppressiven Makrolids aus der Limus-Familie umfasst, enthaltend Partikel mindestens eines pharmazeutisch akzeptablen Hilfsstoffs, auf denen feine kristalline Partikel eines immunsuppressiven Makrolids aus der Limus-Familie gefangen sind.

2. Pharmazeutische Zusammensetzung nach Anspruch 1, wobei die Hilfsstoff-/Produkt-Formulierung kein oberflächenmodifizierendes Mittel und kein Lösungsmittel enthält.

3. Pharmazeutische Zusammensetzung nach Anspruch 1 oder 2, wobei das immunsuppressive Makrolid aus der Limus-Familie aus der Gruppe ausgewählt ist, die den Tacrolimus und den Sirolimus enthält.

4. Pharmazeutische Zusammensetzung nach einem der Ansprüche 1 bis 3, wobei der Hilfsstoff aus der Gruppe ausgewählt ist, die vor allem einen Zucker, ein Polyol, Stärke, ein Zellulosederivat, Magnesiumaluminumsilicat oder deren Gemische enthält.

5. Pharmazeutische Zusammensetzung nach Anspruch 4, wobei der Hilfsstoff aus der Gruppe ausgewählt ist, die vor allem die Laktose, die mikrokristalline Zellulose, das Mannitol und deren Gemische enthält.

6. Pharmazeutische Zusammensetzung nach Anspruch 4 oder 5, wobei der Belastungsgrad des immunsuppressiven Makrolids aus der Limus-Familie auf pharmazeutisch akzeptablen Hilfsstoffpartikeln zwischen 0,5 und 10 % und vorzugsweise zwischen 1 und 4 % inklusive beträgt.

7. Pharmazeutische Zusammensetzung nach einem der Ansprüche 1 bis 6 in Kapsel- oder Tablettenform.

8. Pharmazeutische Zusammensetzung nach Anspruch 7 in Tablettenform, gebildet aus einer Hilfsstoff-/ Produkt-Formulierung eines immunsuppressiven Makrolids aus der Limus-Familie, enthaltend Partikel mindestens eines pharmazeutisch akzeptablen Hilfsstoffs, auf denen feine kristalline Partikel eines immunsuppressiven Makrolids aus der Limus-Familie gefangen sind, und optional einen oder mehrere Hilfsstoffe, die aus der Gruppe ausgewählt sind, die von den Verdünnungsmitteln, den Bindemitteln, den antistatischen Mitteln, den Schmiermitteln, den Permeabilitätsmitteln, den pH verändernden Mitteln, den Antioxidationsmitteln, den Süßstoffen, den Aromen und deren Gemischen ausgewählt sind.

9. Pharmazeutische Zusammensetzung nach einem der Ansprüche 1 bis 8 für seine Verwendung als präventive und/oder kurative Immunsuppressivbehandlung, insbesondere zur Behandlung und/oder Vorbeugung einer Abstoßung nach einer Organtransplantation, beispielsweise der Niere.

10. Herstellungsverfahren feiner kristalliner Partikel von immunsuppressivem Makrolid aus der Limus-Familie, umfassend die folgenden Schritte:
- Solubilisierung eines immunsuppressiven Makrolids aus der Limus-Familie in CO₂, das auf einen Druck über seinem kritischen Druck (7,4 MPa) zwischen 10 und 100 MPa und vorzugsweise zwischen 10 und 40 MPa und auf eine Temperatur über seiner kritischen Temperatur (31 °C), vorzugsweise zwischen 35 und 80 °C, in einem Extraktionsautoklav (5) gebracht wurde,
- plötzlicher Druckabbau der in vorangegangenem Schritt erhaltenen Lösung durch eine Düse (8) in einer Zerstäubereinheit (7), wobei der Druck in der Zerstäubereinheit (7) deutlich über dem atmosphärischen Druck ist, vorzugsweise zwischen 1 und 7 MPa und vorzugsweise zwischen 1 und 4 MPa.

11. Herstellungsverfahren einer Hilfsstoff-/ Produkt-Formulierung, umfassend die folgenden Schritte:
- Solubilisierung eines immunsuppressiven Makrolids aus der Limus-Familie in CO₂, das auf einen Druck über seinem kritischen Druck (7,4 MPa) zwischen 10 und 100 MPa und vorzugsweise zwischen 10 und 40 MPa und auf eine Temperatur über seiner kritischen Temperatur (31 °C), vorzugsweise zwischen 35 und 80 °C, in einem Extraktionsautoklav (5) gebracht wurde,
- plötzlicher Druckabbau der in vorangegangenem Schritt erhaltenen Lösung durch eine Düse (8) in einer Zerstäubereinheit (7), wobei der Druck in der Zerstäubereinheit (7) deutlich über dem atmosphärischen Druck ist, vorzugsweise zwischen 1 und 7 MPa und vorzugsweise zwischen 1 und 4 MPa.
- Fangen der feinen kristallinen Partikel des immunsuppressiven Makrolids aus der Limus-Familie, die sich auf einem Bett eines pharmazeutisch akzeptablen Hilfsstoffs (10) in der Zerstäubereinheit (7) gebildet haben.

## Claims

1. A pharmaceutical composition for orally administering **characterized in that** it comprises an excipient/compound formulation of a "limus" family immunosuppressive macrolide containing particles of at least one pharmaceutically acceptable excipient on which fine crystalline particles of a "limus" family immunosuppressive macrolide are captured.

2. The pharmaceutical composition according to claim 1, wherein the excipient/compound formulation is free of surface modifying agent and solvent.

3. The pharmaceutical composition according to claim 1 or 2, wherein the "limus" family immunosuppressive macrolide is selected from the group comprising tacrolimus and sirolimus.

4. The pharmaceutical composition according to any one of claims 1 to 3, wherein the excipient is selected from the group comprising notably a sugar, a polyol, starch, a cellulose derivative, magnesium aluminosilicate or mixtures thereof.

5. The pharmaceutical composition according to claim 4, wherein the excipient is selected from the group comprising notably lactose, microcrystalline cellulose, mannitol and mixtures thereof.

6. The pharmaceutical composition according to claim 4 or 5, wherein the load of the "limus" family immunosuppressive macrolide on pharmaceutically acceptable excipient particles is between 0.5% and 10%, and preferentially between 1% and 4%.

7. The pharmaceutical composition according to any one of claims 1 to 6, in gelatin capsule form or tablet form.

8. The pharmaceutical composition according to claim 7, in tablet form consisting of an excipient/compound formulation of a "limus" family immunosuppressive macrolide containing particles of at least one pharmaceutically acceptable excipient on which fine crystalline particles of a "limus" family immunosuppressive macrolide are captured and optionally one or more excipients selected from the group consisting of diluents, binders, antistatic agents, lubricants, permeabilizing agents, pH modifiers, antioxidants, sweeteners, flavorings and mixtures thereof.

9. The pharmaceutical composition according to any one of claims 1 to 8, for use as a preventive and/or curative immunosuppressive treatment, in particular to treat and/or prevent rejection after an organ transplant, for example a kidney transplant.

10. A method for preparing fine crystalline particles of "limus" family immunosuppressive macrolide comprising the following steps:
- solubilization of a "limus" family immunosuppressive macrolide in CO₂, carried at a pressure greater than its critical pressure (7.4 MPa), between 10 MPa and 100 MPa, and preferably between 10 MPa and 40 MPa, and at a temperature greater than its critical temperature (31 °C), preferably between 35 °C and 80 °C, in an extraction autoclave (5),
- sudden depressurization of the solution obtained in the preceding step through a nozzle (8) in a spraying enclosure (7), wherein the pressure in the spraying enclosure (7) is substantially greater than atmospheric pressure, preferably between 1 MPa and 7 MPa, and more preferably between 1 MPa and 4 MPa.

11. A method for preparing an excipient/compound formulation comprising the following steps:
- solubilization of a "limus" family immunosuppressive macrolide in CO₂, carried at a pressure greater than its critical pressure (7.4 MPa), between 10 MPa and 100 MPa, and preferably between 10 MPa and 40 MPa, and at a temperature greater than its critical temperature (31 °C), preferably between 35 °C and 80 °C, in an extraction autoclave (5),
- sudden depressurization of the solution obtained in the preceding step through a nozzle (8) in a spraying enclosure (7), wherein the pressure in the spraying enclosure (7) is substantially greater than atmospheric pressure, preferably between 1 MPa and 7 MPa, and more preferably between 1 MPa and 4 MPa,
- capture of the fine crystalline particles of the "limus" family immunosuppressive macrolide formed on a bed of pharmaceutically acceptable excipient (10) placed in the spraying enclosure (7).
